(19) Europäisches Patentamt
European Patent Office
Office européen des brevets

(11) Veröffentlichungsnummer: **0 235 660**
**A1**

(12) **EUROPÄISCHE PATENTANMELDUNG**

(21) Anmeldenummer: **87102176.2**

(22) Anmeldetag: **16.02.87**

(51) Int. Cl.⁴: **C 07 D 249/20**

(30) Priorität: **27.02.86 DE 3606255**

(43) Veröffentlichungstag der Anmeldung: **09.09.87 Patentblatt 87/37**

(84) Benannte Vertragsstaaten: **BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Heitkämper, Peter, Dr., Fliederweg 14, D-4047 Dormagen 11 (DE)**

(54) **Isocyanato-benzotriazole und ein Verfahren zu ihrer Herstellung.**

(57) Neue Isocyanato-benzotriazole der allgemeinen Formel

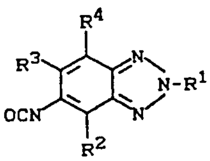

in welcher
$R^1$ für einen aromatischen Kohlenwasserstoffrest steht, der gegebenenfalls mit einer Isocyanatgruppe substituiert ist und/oder gegebenenfalls weitere, inerte Substituenten aufweist,
$R^2$, $R^3$ und $R^4$ für gleiche oder verschiedene Reste stehen und Wasserstoff oder inerte Substituenten bedeuten,
sowie ein Verfahren zur Herstellung der neuen Isocyanate durch Phosgenierung der ihnen zugrundeliegenden Amino-benzotriazole bzw. ihrer Addukte mit Chlorwasserstoff oder mit Kohlendioxid.

EP 0 235 660 A1

0235660

BAYER AKTIENGESELLSCHAFT 5090 Leverkusen, Bayerwerk
Konzernverwaltung RP
Patentabteilung Wr/cm/c

# Isocyanato-benzotriazole und ein Verfahren zu ihrer Herstellung

Die Erfindung betrifft neue Isocyanato-benzotriazole und ein Verfahren zu ihrer Herstellung durch Umsetzung der ihnen zugrundeliegenden Amino-benzotriazole oder von deren Addukten mit Chlorwasserstoff oder Kohlendioxid mit Phosgen.

Von verschiedenen mit Isocyantgruppen substituierten Stickstoff-Heterocyclen ist bekannt, daß sie nicht beständig sind und sich durch Cyclodimerisierung stabilisieren. Dementsprechend führt eine Umsetzung der zugrundeliegenden Amine mit Phosgen auch unmittelbar zu den Dimerisaten (s. zum Beispiel Angewandte Chemie, Band 80, Jahrgang 1968, Seiten 362 bis 364).

Es wurde nun überraschend gefunden, daß sich 2-Aryl-5-amino-benzo-1,2,3-triazole oder deren Addukte mit Chlorwasserstoff oder Kohlendioxid glatt mit Phosgen zu den entsprechenden, bisher nicht bekannten Isocyanaten umsetzen lassen. Weiterhin wurde gefunden, daß diese Isocyanate stabil sind und sich gegebenenfalls unzersetzt destillieren lassen.

Gegenstand der Erfindung sind somit Isocyanato-benzotriazole der allgemeinen Formel

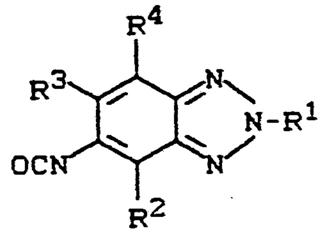

in welcher

$R^1$ für einen aromatischen Kohlenwasserstoffrest steht, der gegebenenfalls mit einer Isocyanatgruppe substituiert ist und/oder gegebenenfalls weitere, inerte Substituenten aufweist,

$R^2$, $R^3$ und $R^4$ für gleiche oder verschiedene Reste stehen und Wasserstoff oder inerte Substituenten bedeuten.

Gegenstand der Erfindung ist auch ein Verfahren zur Herstellung von Isocyanato-benzotriazolen der in Anspruch 1 genannten Formel, dadurch gekennzeichnet, daß man Aminobenzotriazole der allgemeinen Formel

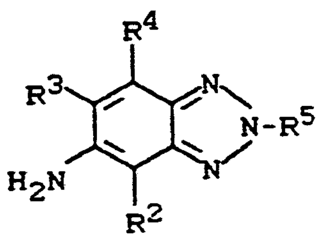

in welcher

$R^2$, $R^3$ und $R^4$ die obengenannten Bedeutungen haben, und

$R^5$ für einen aromatischen Kohlenwasserstoffrest steht, der gegebenenfalls mit einer primären Aminogruppe substituiert ist und/oder der gegebenenfalls weitere, inerte Substituenten aufweist,

oder deren Addukte mit Chlorwasserstoff oder mit Kohlendioxid in an sich bekannter Weise mit Phosgen umsetzt.

Die als Ausgangsmaterialien beim erfindungsgemäßen Verfahren einzusetzenden Amino-benzotriazole der zuletzt genannten allgemeinen Formel sind bekannt und in der Literatur beschrieben (vgl. z.B. Houben-Weyl, Methoden der Organischen Chemie, 4. Auflage, Band 10/3, Seiten 425 ff). Ihre Herstellung gelingt beispielsweise durch Oxidation der entsprechenden 1-Arylazo-2,4-(bzw. 2,5-)-diaminobenzole oder durch Reduktion der entsprechenden 1-Arylazo-2-nitro-4-(bzw. 5-)-amino-benzole.

Als inerte Substituenten $R^2$, $R^3$ oder $R^4$ sowie als inerte Substituenten der aromatischen Kohlenwasserstoffreste $R^1$ und $R^5$ kommen solche Reste in Betracht, die keine Aminogruppe und keine Isocyanatgruppe darstellen und die mit Phosgen oder Isocyanaten nicht reaktiver sind. Beispielhaft seien genannt: Alkylreste, vorzugsweise mit 1 bis 3 Kohlenstoffatomen, Halogen- vorzugsweise Chlor-Substituenten, Alkoxygruppen, vorzugsweise mit 1 bis 3 Kohlenstoffatomen, Alkylthiogruppen, vorzugsweise mit 1 bis 3 Kohlenstoffatomen, Phenyl-, Phenoxy-, Cyano-, Nitro- und/oder Alkoxycarbonylgruppen, vorzugsweise mit insgesamt 2 bis 4 Kohlenstoffatomen.

Bei den bevorzugten, beim erfindungsgemäßen Verfahren einzusetzenden Amino-benzotriazole der zuletzt genannten allgemeinen Formel handelt es sich um solche, für welche

$R^2$, $R^3$ und $R^4$ für gleiche oder verschiedene Reste stehen und Wasserstoff oder einen Alkylrest mit 1 bis 3 Kohlenstoffatomen bedeuten, mit der Maßgabe, daß mindestens 2 dieser Reste für Wasserstoff stehen,

und

$R^5$ für einen Phenyl- oder Naphthyl-Rest steht, der gegebenenfalls eine primäre Aminogruppe als Substituenten und/oder gegebenenfalls einen Alkyl-Substituenten mit 1 bis 3 Kohlenstoffatomen oder einen Chlorsubstituenten aufweist.

Typische Beispiele für beim erfindungsgemäßen Verfahren einzusetzenden Ausgangsamine sind:

5-Amino-2-phenyl-1,2,3-benzotriazol, 5-Amino-2-(4-tolyl)-1,2,3-benzotriazol, 5-Amino-2-(3-ethoxyphenyl)-1,2,3-benzotriazol, 5-Amino-2-(4-nitrophenyl)-1,2,3-benzotriazol, 5-Amino-2-(3-phenoxyphenyl)-1,2,3-benzotriazol, 5-Amino-2-(1-naphthyl)-1,2,3-benzotriazol, 5-Amino-4-methyl-2-phenyl-1,2,3-benzotriazol, 5-Amino-6-methyl-2-phenyl-1,2,3-benzotriazol, 7-Chlor-5-amino-2-phenyl-1,2,3-benzotriazol,

6-Chlor-5-amino-2-(4-methoxy-carbonyl-phenyl)-1,2,3-benzotriazol, 5-Amino-6-isopropyl-2-(4-cyanophenyl)-1,2,3-benzotriazol, 5-Amino-7-methylmercapto-2-(3,4-dimethylphenyl)-1,2,3-benzotriazol, 5-Amino-2-(4-aminophenyl)-1,2,3-benzotriazol, 5-Amino-6-methyl-2-(4-aminophenyl)-1,2,3-benzotriazol, 5-Amino-2-(3-amino-4-methyl-phenyl)-1,2,3-benzotriazol und 5-Amino-6-ethoxycarbonyl-2-(4-aminophenyl)-1,2,3-benztriazol.

Beim erfindungsgemäßen Verfahren können die Amine als solche oder auch in Form ihrer Additionsverbindungen mit Chlorwasserstoff oder mit Kohlendioxid zum Einsatz gelangen. Die erfindungsgemäße Phosgenierung erfolgt im übrigen nach an sich bekannten Methoden, wie sie beispielsweise in Liebigs Annalen der Chemie, Band 562, Jahrgang 1949, Seiten 75 bis 109, in Ullmanns Enzyklopädie der technischen Chemie, Band 14, 4. Auflage, 1977, Seiten 350 bis 354 oder in Houben-Weyl, Methoden der organischen Chemie, Band E4, 4. Auflage, 1983, Seiten 741 bis 753 beschrieben sind.

Die Umsetzung kann kontinuierlich oder diskontinuierlich bevorzugt in Gegenwart eines inerten Lösungsmittels durchgeführt werden. Geeignete Lösungsmittel sind die für Phosgenierungen üblicherweise verwendeten Lösungsmittel, wie aliphatische, cycloaliphatische oder aromatische Kohlenwasserstoffe, Halogenkohlenwasserstoffe, Nitrokohlenwasserstoffe, aliphatisch-aromatische Ether, aromatische Ether, Carbonsäureester, Carbonsäurenitrile, Sulfone, Phosphorsäurehalogenide oder Phosphorsäureester. Als Beispiele für geeignete Lösungsmittel seien genannt:

Trimethylpentan, Decahydronaphthalin, Toluol, 1,2-Dichlorethan, Chlorbenzol, Chlortoluol, 1,2-Dichlorbenzol, Nitrobenzol, Anisol, Phenetol, Diphenylether, Essigsäurebutylester, Tetramethylensulfon, Phosphoroxychlorid, Phosphorsäuretrimethylester. Beliebige Gemische der beispielhaft genannten Lösungsmittel können selbstverständlich ebenfalls eingesetzt werden. Gegebenenfalls weisen die Aminobenzotriazole in einigen der beispielhaft genannten Lösungsmitteln bei niedrigen Temperaturen nur eine geringe Löslichkeit auf. Bei der Durchführung des erfindungsgemäßen Verfahrens erfüllen in solchen Fällen die beispielhaft genannten Lösungsmittel insbesondere bei den niedrigen Phosgeniertemperaturen vor allem die Funktion eines Suspensdiermittels für das Amin bzw. dessen Chlorwasserstoff- oder Kohlendioxid-Addukt und erst bei höheren Temperaturen und mit zunehmender Überführung des Ausgangsmaterials in das Isocyanat die Funktion eines echten Lösungsmittels für das Ausgangsmaterial und insbesondere das Verfahrensprodukt.

Die beim erfindungsgemäßen Verfahren einzusetzenden Gemische aus zu phosgenierendem Ausgangsmaterial und Lösungsmittel haben im allgemeinen einen Gehalt an Amin- bzw. Aminaddukt der oben genannten Art von ca. 2 bis 70 Gew.-%.

Die erfindungsgemäße Phosgenierreaktion kann beispielsweise nach dem bekannten Prinzip der "Kalt-Heiß-Phosgenierung" zweistufig oder nach dem Prinzip der "Heiß-Phosgenierung" einstufig erfolgen. Bei der "Kalt-Heiß-Phosgenierung" erfolgt die Umsetzung des zu phosgenierenden Ausgangsmaterials zu Beginn der Reaktion im allgemeinen bei -20 bis +40° C, vorzugsweise -10 bis +30° C und die anschließende Heißphosgenierung bei 40 bis 260° C, vorzugsweise 80 bis 220° C. Bei dieser "Kalt-Heiß-Phosgenierung" kann der Bereich zwischen der Anfangstemperatur und der erhöhten Temperatur gleichmäßig oder in Sprüngen durchlaufen werden.

Bei der "Heiß-Phosgenierung" kommt das zu phosgenierende Ausgangsmaterial mit dem Phosgen sofort bei Temperaturen von 40 bis 260° C, vorzugsweise 80 bis 220° C in Kontakt.

Die genannte "Kalt-Heiß-Phosgenierung" ist die bevorzugte Verfahrensweise im Falle der Phosgenierung der Amine (anstelle der Chlorwasserstoff- bzw. Kohlendioxid-Addukte).

Bei allen Varianten der erfindungsgemäßen Phosgenierung erfolgt diese vorzugsweise unter Normaldruck oder bei erhöhtem Druck. Der Reaktionsdruck liegt im allgemeinen bei 0,9 bis 100, vorzugsweise 1 bis 60 bar.

Im allgemeinen wird bei der erfindungsgemäßen Phosgenierungsreaktion das zu phosgenierende Ausgangsmaterial mit einer 1- bis 10-fachen, vorzugsweise 1,05- bis 6-fachen stöchiometrischen Menge an Phosgen zusammengebracht. Die genannte Phosgenmenge kann in einer Portion oder auch in Teilmengen in das Reaktionsgemisch eingegeben werden. Es kann vorteilhaft sein, z.B. bei einer diskontinierlichen Arbeitsweise, erst einen Teil der zu verwendenden Phosgenmenge in das Reaktionsgemisch einzubringen und den restlichen Anteil in weiteren Portionen oder stetig über einen

längeren Zeitraum verteilt in das Reaktionsgemisch einzubringen.

Grundsätzlich läßt sich die erfindungsgemäße Phosgenierung der Amine durch Zugabe von Katalysatoren, beispielsweise Dimethylformamid, und/oder Säureakzeptoren, beispielsweise Pyridin, beschleunigen. Im allgemeinen jedoch sind die Reaktionsgeschwindigkeiten bei der Phosgenierung der Amine auch ohne Zugabe eines derartigen Katalysators ausreichend.

Die Reaktionsdauer bei der erfinungsgemäßen Phosgenierung ist von den angewandten Reaktionsbedingungen, insbesondere von den Reaktionstemperaturen, vom Phosgen-Überschuß, von der Verdünnung mit Lösungsmittel und von gegebenenfalls zugegebenen Katalysatoren und/oder Säureakzeptoren abhängig.

Nach Beendigung der Phosgierungsreaktion wird das Reaktionsgemisch in an sich bekannter Weise durch Abtrennung von gasförmigen Bestandteilen (Chlorwasserstoff, überschüssiges Phosgen) und destillativer Entfernung des Lösungsmittels aufgearbeitet. Vor der destillativen Entfernung des Lösungsmittels können gegebenenfalls vorliegende feste Nebenprodukte durch Filtration oder Zentrifugieren entfernt werden. Das nach der destillativen Entfernung des Lösungsmittels als Destillationsrückstand anfallende Rohprodukt kann gewünschtenfalls durch Umkristallisieren aus einem geeigneten inerten Lösungsmittel oder vorzugsweise, falls der Dampfdruck des Isocyanats

dieses gestattet, durch Destillation gereinigt werden. Dabei kann es zweckmäßig sein, die Reindestillation der Isocyanate ohne große Temperaturbelastung, beispielsweise mittels eines Dünnschichtverdampfers durchzuführen. Gewünschtenfalls können aber die erfindungsgemäßen Isocyanate auch durch Tempern bei Temperaturen von 140° C bis 220° C vorzugsweise 160 bis 190° C von störenden Nebenprodukten, beispielsweise thermolabilen, chlorhaltigen Verbindungen befreit werden.

Die erfindungsgemäßen Isocyanate stellen interessante Ausgangsmaterialien zur Herstellung von Pharmazeutika, Schädlingsbekämpfungsmitteln oder Farbstoffen dar. Die erfindungsgemäßen Diisocyanate eignen sich darüber hinaus zur Herstellung von Polyurethankunststoffen, nach dem Isocyanat-Polyadditionsverfahren. Wegen ihres hohen Gehalts an Stickstoff verleihen sie den aus ihnen hergestellten Polyurethanen ein verbessertes Brandverhalten.

In den nachfolgenden Beispielen beziehen sich alle Prozentangaben auf Gewichtsprozente.

0235660

Beispiel 1 (Herstellung von 5-Isocyanato-2-phenyl-1,2,3-benzotriazol

In einer 6 l-Laborphosgenierapparatur wurde eine Lösung von 300 g Phosgen in 3,5 l wasserfreiem Chlorbenzol vorgelegt und bei Raumtemperatur unter Rühren mit 420 g 5-Amino-2-phenyl-1,2,3-benzotriazol versetzt. Das Gemisch wurde unter Rühren zügig auf 60°C erwärmt und dann unter weiterem Einleiten von Phosgen (ca. 50 g/h) im Verlauf von 2 Stunden zum Rückfluß (128°C) erhitzt. Dann wurde das Reaktionsgemisch eine weitere Stunde am Rückfluß phosgeniert. Anschließend wurde die klare Reaktionslösung durch Destillation zuerst bei Normaldruck, schließlich im Wasserstrahlvakuum von überschüssigem Phosgen und vom Chlorbenzol befreit. Das zurückbleibende, kristalline Rohprodukt (482 g) wurde durch Vakuumdestillation gereinigt. Das Isocyanat destillierte bei 160 bis 164°C und 0,1 mbar als farblose Flüssigkeit über, die beim Abkühlen spontan kristallisierte.

Ausbeute: 461 g (98 % d. Th.)
farblose Kristalle vom Schmelzpunkt 138 - 139°C
$C_{13}H_8N_4O$ (236,2)
NCO-Gehalt ber. 17,8 % gef. 17,7 %
Gehalt an hydrolysierbarem Chlor: 105 ppm.

Als Derivat wurde aus dem Isocyanat durch Umsetzung mit Ethanol das entsprechende Ethylurethan hergestellt:

5-(Ethoxycarbonylamino)-2-phenyl-1,2,3-benzotriazol, farblose Kristalle vom Schmelzpunkt 151 - 152°C.

Beispiel 2 (Herstellung von 5-Isocyanato-2-(4-isocyanatophenyl)-1,2,3-Benzotriazol

In einer 6 l-Laborphosgenierapparatur wurde eine Lösung von 400 g Phosgen in 3,5 l wasserfreiem 1,2-Dichlorbenzol vorgelegt und bei Raumtemperatur unter Rühren mit 338 g 5-Amino-2-(4-aminophenyl)-1,2,3-benzotriazol versetzt. Das Gemisch wurde unter Rühren zügig auf 70°C erwärmt und dann unter Einleiten von weiterem Phosgen (ca. 50 g/h) im Verlauf von 3 Stunden zum Rückfluß (176°C) erhitzt. Das Reaktionsgemisch wurde dann eine weitere Stunde am Rückfluß phosgeniert und anschließend durch Andestillieren (ca. 0,5 l) von überschüssigem Phosgen befreit. Zur Abtrennung eines geringen Anteils an Festprodukt wurde das heiße Gemisch filtriert und schließlich auf Raumtemperatur abgekühlt. Das dabei ausfallende Diisocyanat wurde abgesaugt, mit Dichlorbenzol und Petrolether gewaschen und im Vakuum getrocknet. Beim Einengen der Mutterlauge wurde zusätzliches Produkt erhalten. Die vereinigten Rohprodukte wurden aus wasserfreien 1,2-Dichlorbenzol umkristallisiert und ergaben 366 g (88 % d. Th.) sandfarbene Kristalle vom Schmelzpunkt 204 bis 208°C.

$C_{14}H_7N_5O_2$ (277,3)
NCO-Gehalt ber. 30,3 % gef. 29,9 %
Gehalt an hydrolysierbarem Chlor 0,081 %

## Patentansprüche

1. Isocyanato-benzotriazole der allgemeinen Formel

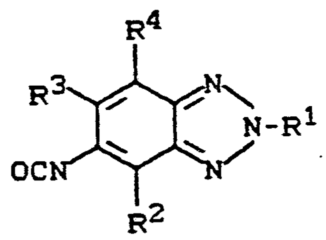

in welcher

$R^1$ für einen aromatischen Kohlenwasserstoffrest steht, der gegebenenfalls mit einer Isocyanatgruppe substituiert ist und/oder gegebenenfalls weitere, inerte Substituenten aufweist,

$R^2$, $R^3$ und $R^4$ für gleiche oder verschiedene Reste stehen und Wasserstoff oder inerte Substituenten bedeuten.

2. Verfahren zur Herstellung von Isocyanato-benzotriazolen der in Anspruch 1 genannten Formel, dadurch gekennzeichnet, daß man Amino-benzotriazole der allgemeinen Formel

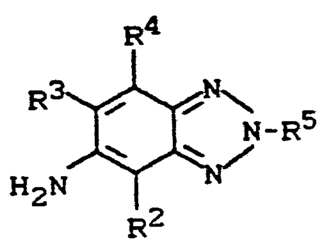

in welcher

$R^2$, $R^3$ und $R^4$ die in Anspruch 1 genannten Bedeutung haben, und

$R^5$ für einen aromatischen Kohlenwasserstoffrest steht, der gegebenenfalls mit einer primären Aminogruppe substituiert ist und/oder der gegebenenfalls weitere, inerte Substituenten aufweist,

oder deren Addukte mit Chlorwasserstoff oder mit Kohlendioxid in an sich bekannter Weise mit Phosgen umsetzt.

Europäisches Patentamt

**EUROPÄISCHER RECHERCHENBERICHT**

0235660

Nummer der Anmeldung

EP 87 10 2176

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | DE-A-2 120 851 (CZESKOSLOVENSKA AKADEMIE VED. PRAG)<br>* Insgesamt *<br>--- | 1,2 | C 07 D 249/20 |
| Y | JOURNAL OF THE CHEMICAL SOCIETY, Band C, Nr. 10, 1966, Seiten 971-976, London, GB; A.J. BOULTON et al.: "N-oxides and related compounds. Part XXVIII. 5-amino- and 5-hydroxy-benzofuroxans"<br>* Insgesamt; insbesondere Seite 975, Verbindung XI *<br>----- | 1 | |
| | | | RECHERCHIERTE SACHGEBIETE (Int. Cl.4) |
| | | | C 07 D 249/00 |

Der vorliegende Recherchenbericht wurde für alle Patentansprüche erstellt.

| Recherchenort | Abschlußdatum der Recherche | Prüfer |
|---|---|---|
| DEN HAAG | 20-06-1987 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82